(19) Europäisches Patentamt
European Patent Office
Office européen des brevets

(11) Numéro de publication: **0 374 014 A2**

(12) **DEMANDE DE BREVET EUROPEEN**

(21) Numéro de dépôt: **89403390.1**

(51) Int. Cl.5: **C07F 13/00, C07C 45/00**

(22) Date de dépôt: **07.12.89**

(30) Priorité: **13.12.88 FR 8816356**

(43) Date de publication de la demande: **20.06.90 Bulletin 90/25**

(84) Etats contractants désignés: **AT BE CH DE ES GB GR IT LI LU NL SE**

(71) Demandeur: **SOCIETE NATIONALE ELF AQUITAINE (PRODUCTION)**
**Tour Elf 2, Place de la Coupole La Défense 6**
**F-92400 Courbevoie(FR)**

(72) Inventeur: **Cahiez, Gérard**
**68 Rue de Turbigo**
**F-75003 Paris(FR)**
Inventeur: **Laboue, Blandine Service M. Cahiez - Univers.P.**
**etM Curie Tour 44-45, 2è étage - 4 Place Jussieu**
**F-75252 Paris Cédex 05(FR)**
Inventeur: **Tozzolino, Pierre**
**Chemin Cerrerot Serres-Morlaas**
**F-64160 Morlaas(FR)**

(74) Mandataire: **Kohn, Armand**
**5 Avenue Foch**
**F-92380 Garches(FR)**

(54) **Nouveaux composés organo-manganeux, leur préparation et applications.**

(57) Composé du manganese bivalent complexé avec un sel d'ammonium quaternaire ; il est préparé par l'action d'un sel d'ammonium quaternaire sur un sel de $Mn^{++}$. surtout halogénure, au sein d'un solvant organique. Le produit convient à la préparation de composés organo-manganeux, à partir desquels on peut synthétiser différents produits chimiques par l'action de composés appropriés, en particulier halogénures d'acide ou anhydrides.

EP 0 374 014 A2

# Nouveaux composés organo-manganeux, leur préparation et applications.

L'invention se rapporte à une nouvelle série de dérivés organiques du manganèse divalent ; elle comprend un procédé pour leur production, ainsi que des applications à la synthèse de cétones.

Au cours de la dernière décennie, les organo-manganeux se sont imposés dans la synthèse chimique, permettant de préparer différents corps commodément, avec de bons rendements. Tels sont, par exemple, les cas des alcools, des acides carboxyliques ou des cétones dont un grand nombre peuvent être produits par l'intermédiaire de composés organo-manganeux au sein d'un solvant. Divers organo-manganeux sont employés, principalement des mixtes, RMnX, où R est un groupe organique et X le plus souvent un halogène ; mais on utilise également des produits du type $R_2Mn$, $R_3MnLi$, $R_3MnMgX$, $R_4MnLi_2$, $R_4Mn(MgX)_2$, etc.

Toutefois, la préparation des organo-manganeux tels que RMnBr et RMnCl par la réaction de l'halogénure de manganèse correspondant, avec un organo-magnésien voire avec un organolithien, se heurte à certaines difficultés, en raison de l'insolubilité de ces halogénures dans les solvants les plus courants, tels que le tétrahydrofuranne ou l'éther diéthylique. Jusqu'à ces dernières années, par exemple, les organo-manganeux mixtes bromés et chlorés n'ont pas pu être préparés quantitativement dans l'éther et, de ce fait, toutes réactions, ultérieures telles que l'acylation de ces composés, conduisaient à des rendements moyens en cétones. D'ailleurs, dans le tétrahydrofuranne,on est obligé d'employer un sel complexe soluble $MnCl_2$,nLiCl pour effectuer la reaction avec RMgX,afin de former le RMnCl voulu. Cependant, l'utilisation des organo-manganeux ainsi préparés dans le tétrahydro furanne n'a pas permis jusqu'à maintenant d'effectuer convenablement certaines réactions.

Ainsi, lors de la réaction d'un anhydride mixte avec un organo-manganeux chloré dans le tétrahydrofuranne, on constate qu'il se forme en majeure partie de l'ester au lieu de la cétone attendue.

La présente invention élargit le champ des possibilités des organo-manganeux par la découverte d'une nouvelle classe de composés de manganèse et de leurs dérivés organiques. Elle permet notamment de résoudre, de façon simple,les problèmes posés par l'insolubilité des halogénures de manganèse dans les solvants courants et par la réactivité insuffisante des organo-manganeux correspondants.

Les nouveaux composés suivant l'invention sont des composés manganeux, caractérisés en ce qu'ils sont complexés avec des sels d'ammoniums quaternaires. Ils peuvent être représentés par la formule générale

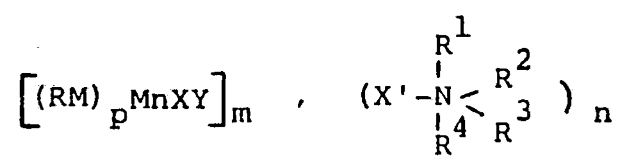

..... (1)

dans laquelle Y et X sont des éléments ou groupes pouvant être des anions, identiques ou différents, ou bien X ou/et Y sont constitués par des groupes hydrocarbonés; X′ est un anion monovalent ; R à $R^3$, semblables ou différents, sont des alkyles ou/et alcényles et/ou aryles en $C_1$ à $C_{18}$, alors que $R^4$ est un alkyle ou alcényle en $C_1$ à $C_{20}$, ou bien un cycloalkyle ou aryle, en $C_6$ à $C_{20}$, chacun des groupes hydrocarbonés pouvant porter des substituants aliphatiques,aromatiques, éthyléniques ou certains groupes fonctionnels, $R^4$ peut comporter un nombre d'atomes de C supérieur à celui de chacun des $R^1$ à $R^3$ ; m est un nombre de 1 à 3, de préférence 1, n étant un nombre de 1 à 10 et de préférence 1 ou 2 ; M est Li ou MgX″, X″ étant un halogène, p étant 0, 1 ou 2. Ainsi p(RM).m(MnXY) peut être $R_2Mn$, $R_3MnLi$, $R_4MnLi_2$, $R_3MnMgX$ ou $R_4Mn(MX)_2$. $R^1$, $R^2$, $R^3$ et $R^4$ peuvent être identiques. Si p=0 et aucun des X et Y ne représente un groupe hydrocarboné, les $R^1$ à $R^3$ sont des alkyles ou alcényles en $C_1$ à $C_7$ et $R^4$ est un alkyle en $C_1$ à $C_7$ ou un benzyle.

A titre d'exemples non limitatifs, voici quelques unes des constitutions que peut présenter le groupe amnonium quaternaire $X'-N(R^1R^2R^3R^4)$:

chlorure de tétraméthyl ammonium,
chlorure de triméthyl stéaryle ammonium,
chlorure de diméthyl dihexyle amnonium
chlorure de tripropyl décyle ammonium
chlorure de diéthyl butyl benzyle ammonium
chlorure de diméthyl benzyl alkyles en $C_8$ à $C_{18}$ ammonium ("BENZALKONIUM")

chlorure ou bromure de tétrabutyl ammonium
chlorure ou bromure de tributyl benzyl ammonium
bromure de diméthyl lauryl benzyle amnonium
bromure de triméthyl cétyl ammonium
bromure de triméthyl lauryl ammonium
bromure de diéthyl propyl oléyl ammonium
bromure de diméthyl isopropyl eicosyl ammonium

Les composés, dans lesquels Y est un groupe hydrocarboné R, c'est-à-dire les organo-manganeux complexés avec un ammonium quaternaire :

$RMnX.N(R^1R^2R^3R^4)X'$ (3)

peuvent porter en R des groupes très divers, aliphatiques cycloaliphatiques, aryliques, pouvant comporter certains substituants fonctionnels. Comme le complexe de formule (3) est généralement obtenu par l'action d'un organo-métallique dérivé d'un métal plus électropositif que Mn, surtout un magnésien (RMgX) ou lithien (RLi), sur le composé de formule (1) transcrite ici sous la forme :

$MnX_2.N(R^1R^2R^3R^4)X'$ (2)

R peut être tout groupe organique que la technique antérieure permet d'avoir dans un magnésien ou un lithien. Ainsi, en tant qu'exemple seulement, on peut citer les alkyles, alcényles et alcynyles, pouvant porter des substituants. De tels groupes aliphatiques ont le plus souvent 1 à 20 atomes de carbone.

Les composés complexés suivant l'invention, en particulier les composés de type $MnCl_2.N(R^1R^2R^3R^4)X'$ ou $MnBr_2.N(R^1R^2R^3R^4)X'$ sont en général solubles dans certains solvants organiques, notamment dans les éthers utilisés pour les synthèses effectuées à partir des organo-manganeux. Cela permet donc d'utiliser commodément les halogénures de manganèse insolubles dans ces solvants. La présente invention concerne en particulier des solutions partielles de $MnBr_2.N(R^1R^2R^3R^4)Br$ (sous forme de gel homogène épais) dans l'éther diéthylique ou dans d'autres éthers aliphatiques acycliques liquides, ainsi que des solutions de $MnX_2.N(R^1R^2R^3R^4)X'$ (X et X' étant Cl ou Br) dans le tétrahydrofuranne ou d'autres éthers cycliques liquides.

La préparation des produits de formule (2) suivant l'invention consiste à agiter un composé de $Mn^{++}$, de préférence un bromure ou un chlorure, avec un sel d'ammonium quaternaire dans un solvant approprié de ce dernier sel, jusqu'à dissolution complète ou partielle de ces réactifs. Les solvants préférés étant des éthers, notamment l'éther éthylique (dans le cas de $MnBr_2$) ou le tétrahydrofuranne (dans celui de $MnCl_2$) , ces solvants sont anhydres ; on opère en général avec des quantités de réactifs d'environ 0,1 à 2 moles par litre, soit de l'ordre de 0,5 mole/litre. On opère à la température ambiante.

Bien que le rapport équimoléculaire soit recommandable entre le composé du Mn et l'ammonium quaternaire, il peut éventuellement être différent.

La préparation décrite ci-dessus conduit au produit de la formule (2) à l'état partiellement ou totalement dissous dans le solvant choisi. La solution obtenue peut servir au passage à la formule (3) par l'addition d'un organo-métallique dérivé d'un métal plus électropositif que le Mn. Cela peut avoir lieu selon la réaction :

(4) $MnX_2.N(R^1R^2R^3R^4)X' + RMgX$ (ou RLi) $\rightarrow RMnX.N(R^1R^2R^3R^4)X' + MgX_2$ (ou LiX) (formule 3)

Suivant la présente invention, on peut notamment ajouter au complexe $MnBr_2.N(R^1R^2R^3R^4)Br$ à l'état partiellement dissous dans l'éther diéthylique ou dans d'autres éthers aliphatiques acycliques, liquides, un composé organo-métallique, de préférence un organo-magnésien de formule RMgX(X=Cl, Br ou I, de préférence Br) ou un organolithien pour obtenir en suspension ou en solution dans l'éther un composé complexe de RMnBr et de $N(R^1R^2R^3R^4)Br$ (dont la présence stabilise RMnBr). On peut également ajouter au complexe $MnX_2.N(R^1R^2R^3R^4)X'$ (X et X' étant Cl ou Br) dissous dans le tétrahydrofuranne, ou dans d'autres éthers cycliques liquides, un composé organo-métallique de préférence un organo-magnésien de formule RMgX″ (X″=Cl, Br ou I) ou un organolithien pour obtenir une solution dans le tétrahydrofuranne d'un complexe de RMnX (de préférence RMnCl) et de $N(R^1R^2R^3R^4)X'$ (de préférence $N(R^1R^2R^3R^4)Cl$).

Lorsque le composé complexe est en solution ou suspension, celle-ci peut servir directement à réaliser des synthèses par addition du corps que l'on veut faire réagir avec RMnX.

Il y a lieu de noter que - dans le choix du solvant -on peut avoir recours pour la réalisation de ces synthèses organiques à des mélanges d'éthers avec des cosolvants, par exemple esters, comme acétate d'éthyle ou autre, acétonitrile, carbonate d'éthyle, ou certains hydrocarbures aromatiques ou aliphatiques (benzène, toluène,cyclohexane, hexane par exemple) etc., ce choix étant à la portée de l'homme de l'art. On peut les utiliser également dans la préparation des organo-manganeux. Quant au complexe (3), suivant l'invention, il permet de réaliser toutes les synthèses qu'il est connu d'opérer au moyen des organo-manganeux indiqués dans la littérature chimique et rappelés au début de la présente description. Ainsi, par exemple, peut-on l'utiliser à la carbonatation de son groupe R, à la transformation d'aldéhydes ou cétones

en alcools, à l'obtention de cétones par acylation de R , etc. Le principe de ces diverses réactions étant connu dans l'art, il n'y a pas lieu ici de donner le détail de toutes ces réactions, dont certaines sont souvent accélérées par la présence de la partie complexante de l'organo-manganeux.

Il est toutefois nécessaire d'indiquer que les complexes (3) suivant l'invention sont particulièrement efficaces pour effectuer les réactions d'acylation du type $RMnX + R'COZ \rightarrow RCOR' + MnXZ$, le rendement en cétone obtenue pouvant, dans certains cas, être considérablement amélioré : ainsi l'acylation par $R'COCl$,dans de l'éther,d'un RMnBr, obtenu selon l'art antérieur, conduit à des rendements en cétone de l'ordre de 50%, tandis qu'avec un RMnBr complexé avec un ammonium quaternaire suivant l'invention, les rendements peuvent être supérieure à 80%.

Comme on l'a déjà indiqué au début de la présente description, les organo-manganeux, préparés dans le tétrahydrofuranne, ne permettent d'effectuer convenablement dans ce solvant certaines acylations avec des agents acylants, tels que les anhydrides mixtes. Suivant une application de la présente invention, on peut utiliser les complexes de formule (3) pour effectuer, dans le tétrahydrofuranne ou dans d'autres éthers cycliques liquides, des réactions avec les divers agents acylants $R'COZ$ connus tels que les halogénures d'acides organiques $R'COX$ (X= halogène de préférence Cl), les anhydrides d'acides organiques tels que $(R'CO)_2O$, $R'COOCOR''$, $R'COOCOOR''$, $R'$ et $R''$ semblables ou différents pouvant être des groupements organiques tous variés notamment aliphatiques, cycloaliphatiques ou aromatiques, substitués, le cas échéant, par des groupes fonctionnels. Les améliorations apportées par l'invention sont particulièrement nettes dans le cas des anhydrides mixtes $R'COOCOOR''$, les rendements en cétone selon l'état de la technique pouvant passer d'environ 30% (non reproductibles suivant l'état de la technique) à environ 80° suivant la présente invention.

L'invention est illustrée par les exemples non limitatifs qui suivent.

## EXEMPLE 1

### Préparation d'un complexe $MnBr_2$-bromure de tétrabutyl ammonium

Dans 80 ml d'éther éthylique anhydre, on introduit 52 mmoles de $MnBr_2$ anhydre (11,2g) avec 50 mmoles (16,7g) de bromure de tétrabutylammonium $Bu_4NBr$ anhydre. Le mélange, maintenu à la température ambiante, est agité continuellement. Après 3 heures d'agitation, le milieu se présente sous la forme d'un gel homogène épais.

## EXEMPLE 2

### Préparation d'un complexe $MnCl_2$-chlorure de tributyl benzyl ammonium

Dans 100 ml de tétrahydrofuranne anhydre, on mélange 52 mmoles de $MnCl_2$ anhydre (6,6g) avec 63,2 mmoles de chlorure de tributyl benzyl ammonium (20,6g) anhydre.

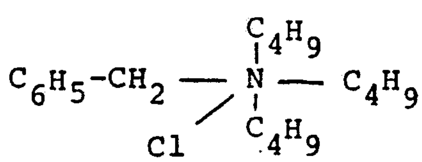

Le tout est agité à la température ambiante, jusqu'à dissolution complète.
On a ainsi obtenu une solution du complexe de $MnCl_2$ avec $ClN(Bu)_3PhCH_2$ dans du THF.

## EXEMPLE 3

### Préparation d'un autre complexe $MnCl_2$-bromure de tétrabutyl ammonium

De la même manière qu'à l'exemple 2, on fait réagir 52 mmoles de $MnCl_2$ anhydre (6,6g) avec 50 mmoles de bromure de tétrabutyl ammonium (16,7 g) dans 80 ml de THF à 20° C. La dissolution complète a eu lieu après 4 heures d'agitation.

EXEMPLE 4

Préparation d'une solution d'organo-manganeux mixte BuMnCl complexé par $Bu_4NBr$

A la solution de 52 mmoles de complexe, dans 100 ml de THF anhydre, obtenu selon l'exemple 3, on ajoute, à 0° C à l'abri de l'air, 50 mmoles de l'organo-magnésien $C_4H_9MgCl$ (ou 50mmoles de BuLi à -30° C) en solution dans 100 ml de THF. Après 15 minutes d'agitation à la température ambiante, on obtient dans les deux cas une solution de BuMnCl complexé par $NBu_4Br$.

EXEMPLE 5

Préparation d'une solution d'organo-manganeux mixte BuMnCl complexé par $ClN(Bu)_3PhCH_2$

A la solution de 52 mmoles de complexe anhydre dans 100ml de THF anhydre, obtenu selon l'exemple 2, on ajoute à 0° C, à l'abri de l'air 50 mmoles de l'organo-magnésien $C_4H_9MgCl$ en solution dans le THF. Après 15 minutes d'agitation à la température ambiante, on obtient une solution de BuMnCl complexé par $ClN(Bu_3)PhCH_2$

EXEMPLE 6

Préparation d'une suspension d'organo-manganeux mixte BuMnBr complexé par $NBu_4Br$

50 mmoles d'organo-magnésien BuMgBr en solution dans l'éther sont ajoutés à 0° C à 52 mmoles du complexe obtenu selon l'exemple 1. Après 1 heure d'agitation, à +10° C, on obtient une suspension marron foncé de BuMnBr complexé par $NBu_4Br$.

EXEMPLE 7

Préparation d'une suspension d'organo-manganeux mixte PhMnBr complexé par $NBu_4Br$

50 mmoles d'organo-magnésien PhMgBr en solution dans l'éther sont ajoutés à 52 mmoles du complexe obtenu selon l'exemple 1. Après 4 heures d'agitation à +10° C, on ob tient une suspension verte de PhMnBr complexé par $NBu_4Br$.

EXEMPLE 8

Application à la production de cétones dans le THF

La préparation de cétones par l'action d'halogénures ou d'anhydrides d'acides, en tant qu'agents acylants, sur des organo-manganeux est connue dans l'art, et elle donne en général de bons résultats. Mais,comme exposé plus haut,des difficultés surgissent surtout lorsque l'agent acylant est un anhydride mixte du type R′COOCOOR″, dont l'emploi serait pourtant intéressant dans certains cas, parce qu'il peut être obtenu dans des conditions douces. On a donc essayé d'acyler $C_4H_9MnCl$ comparativement, au moyen de l'anhydride symétrique de l'acide octanoïque, $(C_7H_{15}CO)_2O$, et à l'aide de l'anhydride mixte

$C_7H_{15}COOCOOC_2H_5$. Dans un cas, l'organo-manganeux BuMnCl est préparé en présence de LiCl et, dans l'autre cas, le même organo-manganeux est préparé selon l'exemple 5.

Dans chacune des quatre opérations, relatées dans le tableau ci-après, on mélangeait 50 mmoles d'anhydride avec 52 mmoles de BuMncl respectivement sous les deux formes susindiquées, dans 100 ml de THF à -30° C. On ramenait ensuite la température à l'ambiante et on agitait le milieu réactionnel durant 2 heures. Le milieu réactionnel était alors traité avec 90 ml de solution aqueuse de HCl 1N : la phase aqueuse décantée était soumise à deux extractions, chacune avec 50 ml de THF. Les phases organiques réunies étaient lavées avec deux fois 50 ml de solution saturée de $K_2CO_3$ dans l'eau. Après séchage sur du $MgSO_4$ anhydre, et évaporation des solutions sous vide,la cétone produite était isolée par distillation. Voici les rendements en pourcentage de butyl heptyl cé tone obtenue dans chacun des quatre essais effectués.

| Composé organo-manganeux traité | Anhydride utilisé | |
|---|---|---|
| | $(C_7H_{15}CO)_2O$ | $C_7H_{15}COOCOOC_2H_5$ |
| BuMnCl avec LiCl | 85 | 30 |
| BuMnCl avec $N(Bu_3PhCH_2)Cl)$ | 86 | 79 |

Ainsi, avec l'anhydride mixte,l'organo-manganeux complexé par LiCl donne seulement 30% de rendement, ce qui confirme les résultats obtenus antérieurement (Tetrahedron vol.40, n° 4, page 686; 1984). Par contre, le complexe avec l'ammonium quaternaire, suivant l'invention, conduit avec cet anhydride à un résultat tout à fait acceptable de 79%, très rapidement.

EXEMPLES 9 à 14

Des acylations analogues à celles de l'exemple 8 ont été effectuées au moyen d'anhydrides mixtes $R'COOCOOC_2H_5$ portant différents groupes R′ sur des complexes organo-manganeux RMnCl avec $ClN(Bu)_3PhCH_2$ dont le groupe R variait d'un essai à l'autre.

Le Tableau ci-après indique la nature des R,R′ et le rendement en cétone RCOR′ obtenue.

| EXEMPLE n° | R | R′ | Rendement % |
|---|---|---|---|
| 9 | Bu | Hept | 79 |
| 10 | Ph | Hept | 68 |
| 11 | Hept | iPr | 78 |
| 12 | Bu | Ph | 85 |
| 13 | iPr | Ph | 79 |
| 14 | Hept | $Me_2C=CH$ | 83 |

Les résultats convenables de l'exemple 8 sont, comme on le voit, confirmé sur six autres cas. A noter qu'exceptionnellement, dans l'essai n° 11, il a fallu abaisser la température pendant l'addition de l'anhydride dans le milieu réactionnel.

EXEMPLE 15

Application à la production de cétones dans l'éther

Comme on l'a déjà exposé précédemment, l'acylation, par R′COCl dans l'éther, de RMnBr préparé par action d'un organo-magnésien sur du bromure de manganèse non complexé, conduit à des rendements très moyens en cétone.

On a essayé d'acyler par BuCOcl, un BuMnBr préparé par la technique antérieure et par un BuMnBr complexé par $Bu_4NBr$ préparé selon l'exemple 5. Dans ces deux opérations, on ajoutait 50 mmoles de

BuCOCl, sous agitation, à 52 mmoles de BuMnBr en suspension dans l'éther à -20 °C. On ramenait ensuite la température à l'ambiante, et on agitait le milieu réactionnel pendant deux heures. Puis on le traitait avec 60 ml de HCl 1N à 10 °C ; le phase aqueuse décantée était soumise à deux extractions, chacune avec 50 ml d'éther. Les phases organiques réunies étaient ensuite lavées avec 50 ml d'une solution saturée de $NaHCO_3$. On isole ensuite la cétone comme dans l'exemple 8. Le rendement obtenu selon l'art antérieur est de 54%,et avec le complexe selon l'invention de 83% en BuCOBr, ce qui met en évidence le rôle déterminant joué par la complexation de $MnBr_2$ lors de la préparation de BuMnBr.

## Revendications

1. Composé du manganèse bivalent, complexé avec un sel d'ammonium quaternaire, caractérisé par la constitution suivante :

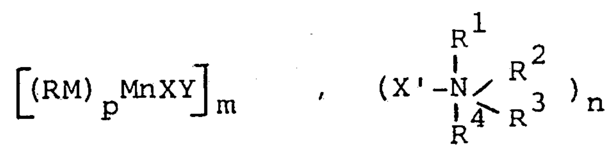

dans laquelle :

a) - p est 0 ; 1 ou 2

- m est 1 ; 2 ou 3, de préférence 1,
- n est compris entre 1 et 10, et est de préférence 1 ou 2

b) - R est un groupe hydrocarboné,

- M est Li ou MgX″ (X″ = Cl, Br ou I)

c) - X et Y sont deux groupements hydrocarbonés, de préférence identiques,

ou - X et Y sont des éléments ou groupes pouvant former deux anions monovalents, de préférence identiques (dans ce cas p = 0)

ou - X est un anion tandis que Y est un groupe hydrocarboné

d) - X′ est un anion monovalent

- $R^1$,$R^2$,$R^3$, semblables ou différents, sont des alkyles ou des alcényles en $C_1$ à $C_{18}$, des cycloalkyles en $C_5$ à $C_{18}$ ou bien des aryles en $C_6$ à $C_{18}$, alors que $R^4$ est un alkyle ou alcényle en $C_1$ à $C_{20}$, ou bien un cycloalkyle $C_5$ à $C_{18}$, ou un aryle en $C_6$ à $C_{20}$, chacun des groupes hydrocarbonés pouvant porter des substituants, aliphatiques saturés, aromatiques, éthyléniques ou des groupes fonctionnels ;
- si p = 0 et aucun des X et Y n'est un groupe hydrocarboné, les $R^1$,$R^2$,$R^3$ sont des alkyles ou alcényles en $C_1$ à $C_7$ et R″ est un alkyle en $C_1$ à $C_7$ ou un benzyle.

2. Composé suivant la revendication 1, caractérisé en ce les groupes $R^1$,$R^2$,$R^3$ et $R^4$ sont identiques.

3. Composé suivant la revendication 1, caractérisé en ce que le groupe $R^4$ comporte un nombre d'atomes de carbone supérieur ou inférieur à celui de chacun des groupes $R^1$ à $R^3$.

4. Composé suivant la revendication 1, caractérisé en ce que X′N($R_1$,$R_2$,$R_3$,$R_4$) est le chlorure de tétrabutyle ammonium, le chlorure de tributyl benzyl ammonium, le bromure de tétrabutyl ammonium ou le bromure de tributyl benzyl ammonium.

5. Composé suivant une des revendications 1 à 4, caractérisé en ce que Y, X et X′ semblables ou différents sont des halogènes, de préférence le chlore et le brome.

6. Composé suivant la revendication 5, caractérisé en ce qu'il est totalement ou partiellement dissous dans un solvant organique, de préférence un éther.

7. Composé suivant la revendication 6, caractérisé en ce que le solvant est un éther acyclique aliphatique liquide de préférence l'éther diéthylique et que Y, X et X′ sont des atomes de brome.

8. Composé suivant la revendication 5, caractérisé en ce que le solvant est un éther cyclique, liquide, de préférence le tétrahydrofuranne, que Y et X sont identiques et sont des atomes de brome ou de chlore et que X′, identique ou différent de X et Y, est un atome de brome ou de chlore.

9. Composé suivant une des revendications 1 à 4, caractérisé en ce que Y étant un groupe hydrocarboné R, celui-ci est aliphatique, cycloaliphatique ou arylique, pouvant comporter certains substituants fonctionnels.

10. Composé suivant la revendication 9, caractérisé en ce que les groupes R sont des alkyles ou alcényles, linéaires ou ramifiés, en $C_1$ à $C_{20}$, qui peuvent porter des substituants.

11. Composé suivant une des revendications 1 à 4, caractérisé en ce que X et X′, semblables ou

différents, sont des halogènes et plus particulièrement des atomes de chlore ou de brome.

12. Composé suivant la revendication 11, caractérisé en ce que X est un atome de chlore, et que $X'$ est un atome de chlore ou de brome.

13. Composé suivant la revendication 11, caractérisé en ce que X et $X'$ sont des atomes de brome.

14. Composé suivant la revendication 9 ou 10, caractérisé en ce que X et Y sont des groupes hydrocarbonés R.

15. Composé suivant la revendication 1, caractérisé en ce que $(RM)_pMnXY$ est $R_3MnLi$, $R_4MnLi_2$, $R_3MnMgX$ ou $R_4Mn(MgX)_2$.

16. Procédé de préparation d'un composé du $Mn^{++}$ complexé avec un sel d'ammonium quaternaire, caractérisé en ce que l'on agite un sel de $Mn^{++}$, de préférence un chlorure ou un bromure, avec un sel d'ammonium quaternaire, au sein d'un solvant de ce dernier, de préférence un éther, jusqu'à dissolution totale ou partielle des deux sels.

17. Procédé suivant la revendication 16, caractérisé en ce que le sel de $Mn^{++}$ est un bromure et que le solvant est un éther aliphatique acyclique de préférence l'éther diéthylique.

18. Procédé suivant la revendication 16, caractérisé en ce que le sel de $Mn^{++}$ est un chlorure ou un bromure et que le solvant est un éther cyclique de préférence le tétrahydrofuranne.

19. Procédé suivant une des revendications 16 à 18, caractérisé en ce que l'on ajoute à la solution obtenue un composé organo-métallique dérivé d'un métal plus électropositif que le manganèse, portant un groupe organique R, et on laisse réagir, sous agitation, jusqu'à ce que ce groupe R soit transféré sur le manganèse du composé complexé.

20. Procédé suivant la revendication 19, caractérisé en ce que le composé organo-métallique est RMgX ou RLi.

21. Procédé suivant la revendication 19 ou 20, caractérisé en ce qu'on ajoute RMgBr ou RLi à la solution obtenue suivant la revendication 17.

22. Procédé selon la revendication 19 ou 20, caractérisé en ce que l'on ajoute RMgCl ou RLi à la solution obtenue suivant la revendication 18.

23. Application du composé de $Mn^{++}$ complexé avec un sel d'ammonium quaternaire à la préparation d'un organo-manganeux RMnX (X étant un anion), caractérisée en ce que l'on réalise le procédé suivant la revendication 19 dans un solvant dans lequel RMnX est soluble ou n'est pas soluble.

24. Application d'un composé de Mn complexé, suivant une des revendications 1 à 4 et 9 à 15, dans lequel Y est un groupe organique, à la synthèse d'un produit chimique par la réaction de ce composé avec un substrat susceptible de fixer le groupe Y.

25. Application suivant la revendication 24, caractérisée en ce qu'elle consiste en la production d'un acide carboxylique, la transformation d'un aldéhyde ou d'une cétone en alcool, à la transformation d'une cétone en énolate ou en une autre cétone.

26. Application suivant la revendication 25, caractérisée en ce que l'on prépare une cétone par l'action de l'organo-manganeux complexé sur un chlorure d'acide organique, un anhydride ou $R'COOCOR''$, ou un anhydride mixte $R'COOCOOR''$, $R'$ et $R''$, semblables ou différents étant des groupes organiques.

27. Application suivant la revendication 26, caractérisée en ce que l'on prépare une cétone par réaction, dans l'éther diéthylique, d'un chlorure d'acide organique avec un composé suivant la revendication 13.

28. Application suivant la revendication 26, caractérisée en ce que l'on prépare une cétone par réaction, dans le tétrahydrofuranne, d'un anhydride mixte avec un composé suivant une des revendications 11 à 13.

Revendications pour l'Etat contractant suivant: ES

1. Procédé de préparation d'un composé organique par la réaction d'un organo-manganeux avec un composé organique de départ, porteur d'un atome ou groupe susceptible de réagir avec l'organo-manganeux, caractérisé en ce que celui-ci est utilisé sous la forme d'un complexe avec un sel d'ammonium quaternaire répondant à la formule

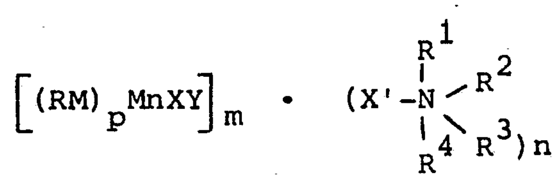

dans laquelle R est un groupe hydrocarboné, M est Li ou $MgX''$ ($X''$ étant Cl, Br ou I) ; p est 0 à 2 ; m = 1 à 3 et n = 1 à 10 ; chacun des X et Y, semblables ou différents, est un anion monovalent ou un groupe

hydrocarboné, un au moins d'entre eux étant un tel groupe, lorsque p = 0 ; X′ est un anion monovalent ; $R^1$,$R^2$,$R^3$, semblables ou différents, sont des alkyles ou des alcényles en C à $C_{18}$, de cycloalkyles en $C_5$ à $C_{18}$ ou bien des aryles en $C_6$ à $C_{18}$, alors que $R^4$ est un alkyle ou alcényle en $C_1$ à $C_{20}$, un cycloalkyle en $C_5$ à $C_{18}$ ou un aryle en $C_6$ à $C_{20}$, chacun de ces groupes hydrocarbonés pouvant porter des substituants aliphatiques saturés, aromatiques, éthyléniques, ou des groupes fonctionnels.

2. Procédé suivant la revendication 1, caractérisé en ce que les groupes $R^1$,$R^2$,$R^3$ et $R^4$ sont identiques.

3. Procédé suivant la revendication 1, caractérisé en ce que le groupe $R^4$ comporte un nombre d'atomes de carbone supérieur ou inférieur à celui de chacun des groupes $R^1$ à $R^3$.

4. Procédé suivant la revendication 1, caractérisé en ce que X′N($R_1$,$R_2$,$R_3$,$R_4$) est le chlorure de tétrabutyle am monium, le chlorure de tributyl benzyl ammonium, le bromure de tétrabutyl ammonium ou le bromure de tributyl benzyl amnonium.

5. Procédé suivant une des revendications 1 à 4, caractérisé en ce que Y, X et X′ semblables ou différents sont des halogènes, de préférence le chlore et le brome.

6. Procédé suivant la revendication 5, caractérisé en ce que le complexe d'organo-manganeux est totalement ou partiellement dissous dans un solvant organique, de préférence un éther.

7. Procédé suivant la revendication 6, caractérisé en ce que le solvant est un éther acyclique aliphatique, liquide, de préférence l'éther diéthylique, et que Y, X et X′ sont des atomes de brome.

8. Procédé suivant la revendication 5, caractérisé en ce que le solvant est un éther cyclique, liquide, de préférence le tétrahydrofuranne, que Y et X sont identiques et sont des atomes de brome ou de chlore, et que X′, identique ou différent de X et Y, est un atome de brome ou de chlore.

9. Procédé suivant une des revendications 1 à 4, caractérisé en ce que Y étant un groupe hydrocarboné R, celui-ci est aliphatique, cycloaliphatique ou arylique, pouvant comporter des substituants fonctionnels.

10. Procédé suivant la revendication 9, caractérisé en ce que les groupes R sont des alkyles ou alcényles, linéaires ou ramifiés, en $C_1$ à $C_{20}$, qui peuvent porter des substituants.

11. Procédé suivant une des revendications 1 à 4, caractérisé en ce que X et X′, semblables ou différents, sont des halogènes et plus particulièrement des atomes de chlore ou de brome.

12. Procédé suivant la revendication 11, caractérisé en ce que X est un atome de chlore et que X′ est atome de chlore ou de brome.

13. Composé suivant la revendication 9 ou 10, caractérisé en ce que X et Y sont des groupes hydrocarbonés R.

14. Composé suivant la revendication 1, caractérisé en ce que $(RM)_pMnXY$ est $R_3MnLi$, $R_4MnLi_2$, $R_3MnMgX$ ou $R_4Mn(MgX)_2$.

15. Procédé suivant une des revendications précédentes, dans lequel le composé du $Mn^{++}$, complexé avec un sel d'ammonium quaternaire, a été préparé par l'agitation d'un composé de $Mn^{++}$, de préférence chloré ou bromé, avec un sel d'ammonium quaternaire, au sein d'un solvant de ce dernier, de préférence un éther, jusqu'à dissolution totale ou partielle des deux sels.

16. Procédé suivant la revendication 15, caractérisé en ce qu'à la solution obtenue a été ajouté un composé organo-métallique dérivé d'un métal plus électropositif que le manganèse, portant un groupe organique R, et on a laissé réagir, sous agitation, jusqu'à ce que ce groupe R fut transféré sur le manganèse du composé complexé.

17. Procédé suivant la revendication 16, caractérisé en ce que le composé organo-métallique est RMgX ou RLi.

18. Procédé suivant une des revendications précédentes, caractérisé en ce qu'il consiste en la production d'un acide carboxylique, la transformation d'un aldéhyde ou d'une cétone en alcool, à la transformation d'une cétone en énolate ou bien en une autre cétone.

19. Procédé suivant la revendication 18, caractérisé en ce que l'on prépare une cétone par l'action de l'organo-manganeux complexé sur un chlorure d'acide organique, sur un anhydride ou R′COOCOR″, ou sur un anhydride mixte R′COOCOOR″, R′ et R″, semblables ou différents étant des groupes organiques.

20. Procédé suivant la revendication 19, caractérisé en ce que l'on prépare une cétone par réaction, dans l'éther diéthylique, d'un chlorure d'acide organique avec un complexe manganeux suivant la revendication 1, dans lequel X et X′ sont des Br.

21. Procédé suivant la revendication 19, caractérisé en ce que l'on prépare une cétone par réaction, dans le tétrahydrofuranne, d'un anhydride mixte avec un complexe manganeux suivant la revendication 1, dans lequel X et X′ sont des atomes de brome.

Revendications pour l'Etat contractant suivant: AT

1. Procédé de préparation d'un composé organique par la réaction d'un organo-manganeux avec un

composé organique de départ, porteur d'un atome ou groupe susceptible de réagir avec l'organo-manganeux, caractérisé en ce que celui-ci est utilisé sous la forme d'un complexe avec un sel d'ammonium quaternaire répondant à la formule

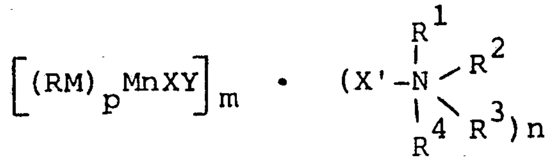

dans laquelle R est un groupe hydrocarboné, M est Li ou MgX″ (X″ étant Cl, Br ou I) ; p est 0 à 2 ; m = 1 à 3 et n = 1 à 10 ; chacun des X et Y, semblables ou différents, est un anion monovalent ou un groupe hydrocarboné, un au moins d'entre eux étant un tel groupe, lorsque p = 0 ; X′ est un anion monovalent ; $R^1$,$R^2$,$R^3$, semblables ou différents, sont des alkyles ou des alcényles en $C_1$ à $C_{18}$, de cycloalkyles en $C_5$ à $C_{18}$ ou bien des aryles en $C_6$ à $C_{18}$, alors que $R^4$ est un alkyle ou alcényle en $C_1$ à $C_{20}$, un cycloalkyle en $C_5$ à $C_{18}$ ou un aryle en $C_6$ à $C_{20}$, chacun de ces groupes hydrocarbonés pouvant porter des substituants aliphatiques saturés, aromatiques, éthyléniques, ou des groupes fonctionnels.

2. Procédé suivant la revendication 1, caractérisé en ce que les groupes $R^1$,$R^2$,$R^3$ et $R^4$ sont identiques.

3. Procédé suivant la revendication 1, caractérisé en ce que le groupe $R^4$ comporte un nombre d'atomes de carbone supérieur ou inférieur à celui de chacun des groupes $R^1$ à $R^3$.

4. Procédé suivant la revendication 1, caractérisé en ce que X′N($R_1$,$R_2$,$R_3$,$R_4$) est le chlorure de tétrabutyle am monium, le chlorure de tributyl benzyl ammonium, le bromure de tétrabutyl ammonium ou le bromure de tributyl benzyl ammonium.

5. Procédé suivant une des revendications 1 à 4, caractérisé en ce que Y, X et X′ semblables ou différents sont des halogènes, de préférence le chlore et le brome.

6. Procédé suivant la revendication 5, caractérisé en ce que le complexe d'organo-manganeux est totalement ou partiellement dissous dans un solvant organique, de préférence un éther.

7. Procédé suivant la revendication 6, caractérisé en ce que le solvant est un éther acyclique aliphatique, liquide, de préférence l'éther diéthylique, et que Y, X et X′ sont des atomes de brome.

8. Procédé suivant la revendication 5, caractérisé en ce que le solvant est un éther cyclique, liquide, de préférence le tétrahydrofuranne, que Y et X sont identiques et sont des atomes de brome ou de chlore, et que X′, identique ou différent de X et Y, est un atome de brome ou de chlore.

9. Procédé suivant une des revendications 1 à 4, caractérisé en ce que Y étant un groupe hydrocarboné R, celui-ci est aliphatique, cycloaliphatique ou arylique, pouvant comporter des substituants fonctionnels.

10. Procédé suivant la revendication 9, caractérisé en ce que les groupes R sont des alkyles ou alcényles, linéaires ou ramifiés, en $C_1$ à $C_{20}$, qui peuvent porter des substituants.

11. Procédé suivant une des revendications 1 à 4, caractérisé en ce que X et X′, semblables ou différents, sont des halogènes et plus particulièrement des atomes de chlore ou de brome.

12. Procédé suivant la revendication 11, caractérisé en ce que X est un atome de chlore et que X′ est atome de chlore ou de brome.

13. Composé suivant la revendication 9 ou 10, caractérisé en ce que X et Y sont des groupes hydrocarbonés R.

14. Composé suivant la revendication 1, caractérisé en ce que $(RM)_pMnXY$ est $R_3MnLi$, $R_4MnLi_2$, $R_3MnMgX$ ou $R_4Mn(MgX)_2$.

15. Procédé suivant une des revendications précédentes, dans lequel le composé du $Mn^{++}$, complexé avec un sel d'ammonium quaternaire, a été préparé par l'agitation d'un composé de $Mn^{++}$, de préférence chloré ou bromé, avec un sel d'ammonium quaternaire, au sein d'un solvant de ce dernier, de préférence un éther, jusqu'à dissolution totale ou partielle des deux sels.

16. Procédé suivant la revendication 15, caractérisé en ce qu'à la solution obtenue a été ajouté un composé organo-métallique dérivé d'un métal plus électropositif que le manganèse, portant un groupe organique R, et on a laissé réagir, sous agitation, jusqu'à ce que ce groupe R fut transféré sur le manganèse du composé complexé.

17. Procédé suivant la revendication 16, caractérisé en ce que le composé organo-métallique est RMgX ou RLi.

18. Procédé suivant une des revendications précédentes, caractérisé en ce qu'il consiste en la production d'un acide carboxylique, la transformation d'un aldéhyde ou d'une cétone en alcool, à la transformation d'une cétone en énolate ou bien en une autre cétone.

19. Procédé suivant la revendication 18, caractérisé en ce que l'on prépare une cétone par l'action de

l'organo-manganeux complexé sur un chlorure d'acide organique, sur un anhydride ou R′COOCOR″, ou sur un anhydride mixte R′COOCOOR″, R′ et R″, semblables ou différents étant des groupes organiques.

20. Procédé suivant la revendication 19, caractérisé en ce que l'on prépare une cétone par réaction, dans l'éther diéthylique, d'un chlorure d'acide organique avec un complexe manganeux suivant la revendication 1, dans lequel X et X′ sont des Br.

21. Procédé suivant la revendication 19, caractérisé en ce que l'on prépare une cétone par réaction, dans le tétrahydrofuranne, d'un anhydride mixte avec un complexe manganeux suivant la revendication 1, dans lequel X et X′ sont des atomes de brome.